# EUROPEAN PATENT APPLICATION

(11) **EP 4 137 566 A1**
(43) Date of publication of application: **22.02.2023**
(21) Application number: 21776770.6
(22) Date of filing: 24.03.2021
(51) Int. Cl.: C12N 5/10, A01H 5/00, C12N 15/52, C12Q 1/04, A01H 6/46

(54) **ORGANELLE TRANSFORMANT SCREENING METHOD**

(30) Priority: 25.03.2020 JP 2020054225
(71) Applicant: National University Corporation Kobe University, Kobe-shi, Hyogo 657-8501 (JP)
(72) Inventor: KATAYAMA, Kenta, Kobe-shi, Hyogo 657-8501 (JP); TERAMOTO, Jun, Kobe-shi, Hyogo 657-8501 (JP); NISHIDA, Keiji, Kobe-shi, Hyogo 657-8501 (JP)
(74) Representative: Heubeck, Christian
(86) International application number: PCT/JP2021/012142
(87) International publication number: WO 2021/193688

(57) **Abstract**

The present invention provides an organelle transformant screening method. More specifically, the present invention provides an organelle transformant screening method utilizing inhibition of a lipid synthetic pathway.

## Description

### [Technical Field]

The present invention relates to a method of screening organelle transformants. More specifically, the present invention relates to a method of screening organelle transformants using inhibition of a lipid synthesis pathway.

### [Background Art]

Introduction of an exogenous gene into a plant cell to impart a useful trait that cannot be added by cross breeding to a plant, has vital significance in future improvement of agricultural products and advancement in agriculture, such as increased food production or contribution to promoted improvement in the environment during rapid changes in the state of agriculture, such as the recent rapid increase in population or climate change. Technological improvements in genetic engineering of plants are essential in order to materialize production of substances with plants by using carbon dioxide as a raw material, from the viewpoint of limited supplies of fossil fuel, etc.

As one of the technologies for genetic engineering of plant cells, a genetic engineering technology targeting organellar DNA such as a plastid (e.g., chloroplast) or mitochondria, instead of a nucleus, is known (e.g., Non Patent Literature 1). Generally, tens to thousands of organelles are within a cell (e.g., about 100 per cell for chloroplasts), and tens to hundreds of genomes are present in each organelle. Thus, high expression level of a gene introduced into the organelle is expected. In the cytoplasm, even toxic proteins or ribonucleic acids can accumulate without a specific cleavage or modification in each organism in an organelle with no silencing, so that gene expression is also stable in future generations. Furthermore, the organelle is often limited to maternal or paternal inheritance, so that the risk of proliferation of an exogenous gene is extremely low.

As such a method of transforming an organelle, a highly efficient method of transforming a chloroplast is known in single cell organisms such as Chlamydomonas and dicotyledons including asterids such as Nicotiana tabacum. While a method of screening transformants using spectinomycin is exclusively used as a method of transforming a chloroplast in a dicotyledon (Non Patent Literature 1), with a method using spectinomycin, the efficiency of transformation of chloroplasts in plants of the family Brassicaceae such as Arabidopsis thaliana is very low, but a realistic transformation has been finally successful after introducing a mutation in an ACC2 gene (Non Patent Literature 2). Since monocotyledons such as Oryza sativa are highly resistant to spectinomycin, screening using spectinomycin cannot be applied. As far as the inventors are aware, a reproducible method of transforming an organelle in monocotyledons has not been reported.

### [Citation List]

### [Non Patent Literature]

[NPL 1] Day, A. and Goldschmidt-Clermont, M., Plant Biotechnol. J., 9: 540 (2011)
[NPL 2] Yu, Q. et al., Plant Physiol., 175(1) : 186-193 (2017)

### [Summary of Invention]

### [Technical Problem]

Thus, the problem to be solved by the present invention is to provide a method that can efficiently transform an organelle in any plant including monocotyledons, Brassicaceae, etc., and an organelle transformant obtained by said method.

### [Solution to Problem]

The inventors conjectured that one of the reasons transformation was not successful in monocotyledons is that since the ratio of transformed organelles to all organelles contained in a plant cell is low, transformed organelles are exterminated by untransformed organelles, resulting in no remaining transformed organelles. In this regard, the inventors conceived of a method of increasing the ratio of transformed organelles accounted for in a cell by culturing cells under a condition where cells with an increase in organelles other than transformed organelles die and growing only transformed organelles in a plant cell. To achieve such an objective, the inventors focused on lipid-soluble compounds remaining within an organelle that are essential, instead of water-soluble compounds that can move between organelles via the cytoplasm. This is because the inventors considered the potential of acquiring drug resistance, etc., in untransformed organelles when screening using a substance that can move between organelles as an indicator. As such an essential lipid-soluble compound, the inventors focused on fatty acids that can be a raw material for a lipid, which is essential for forming a lipid bilayer such as a thylakoid membrane. By using a compound that inhibits a synthesis system of said fatty acid, the ratio of organelles within a cell was successfully increased by selectively screening for only transformed organelles imparted with resistance to said compound. Furthermore, transformation was successfully performed more efficiently by increasing the ratio of nucleoids having an introduced gene to nucleoids contained in a cell by using the gene drive technology.

ACC2 is an enzyme that is involved with the synthesis of fatty acids in a chloroplast (catalyzes synthesis from acetyl-CoA to malonyl-CoA) in plants of the family Brassicaceae, etc. The synthesis pathway (hereinafter, malonyl-CoA synthesis pathway) in plants of the family Brassicaceae, etc. includes not only a pathway catalyzed by ACC2 (hereinafter, also referred to as "eukaryotic ACC pathway"), but also pathways catalyzed by a CAC1, CAC2, CAC3, and AccD complex (hereinafter, also referred to as "prokaryotic ACC pathway"). Spectinomycin blocks only the latter pathway. For this reason, it was hypothesized that screening efficiency by spectinomycin is low in plants of the family Brassicaceae, etc., which have both pathways in a chloroplast. The inventors conceived a method of efficiently transforming an organelle in plants of the family Brassicaceae, etc. by concomitantly using spectinomycin with an inhibitor for a pathway catalyzed by ACC2.

Furthermore, in view of the findings described above, a gene encoding a protein that compensates for the function of an enzyme associated with a synthesis pathway of glycerolipid, which is essential for the formation of a lipid bilayer of an organelle as another essential lipid-soluble compound, and a gene encoding a protein for production in other organelles are introduced from the outside by using a mutant with the enzymatic activity inactivated from introducing a mutation to the enzyme, and the enzyme and protein are expressed in an organelle to successfully perform transformation in the organelle. The present invention was completed as a result of further studies by the inventors based on these findings.

Specifically, the present invention is the following.
[1] A method of screening for monocotyledon cells with a transformed organelle, comprising the step of contacting an inhibitor of ACC2 with a cell population comprising monocotyledon cells introduced with a gene encoding acetyl-CoA carboxylase having resistance to the inhibitor and a gene encoding a protein of interest into an organelle.
[2] The method of [1], wherein the acetyl-CoA carboxylase is derived from an organism other than the monocotyledon.
[3] The method of [1] or [2], wherein the monocotyledon is a plant of the family Poaceae.
[4] A method of selecting a plant cell with a transformed organelle, comprising the step of contacting an inhibitor of ACC2 and spectinomycin with a cell population comprising plant cells introduced with a gene encoding acetyl-CoA carboxylase having resistance to the inhibitor or a gene resistant to spectinomycin and a gene encoding a protein of interest into an organelle.
[5] The method of any of [1] to [4], wherein the inhibitor of ACC2 is at least one selected from the group consisting of pinoxaden, haloxyfop, tepraloxydim, and tralkoxydim.
[6] The method of any of [1] to [5], comprising the step of increasing a ratio of nucleoids having the introduced gene to nucleoids contained in an organelle by destroying nucleoids other than nucleoids having the introduced gene and selectively replicating nucleoids having the introduced gene, prior to contacting the inhibitor of ACC2 with plant cells.
[7] The method of any of [1] to [6], wherein the organelle is a plastid or a mitochondrion.
[8] A plant cell screened for by the method of any of [1] to [7] or a plant comprising said cell.
[9] A method of manufacturing a plant cell with a transformed organelle, comprising the step of introducing a gene encoding a protein that compensates for a function of an enzyme involved with a synthesis pathway of an essential lipid-soluble compound into an organelle and a gene encoding a protein of interest to an organelle of a plant cell with the function of the enzyme that is attenuated.
[10] The method of [9], wherein the enzyme involved with a synthesis pathway of an essential lipid-soluble compound is selected from a group of enzymes involved with a lipid synthesis pathway.
[11] The method of [9], wherein the enzyme involved with a synthesis pathway of an essential lipid-soluble compound is selected from the group consisting of PGP1, CLS, and MGD1.
[12] A plant cell obtained by the method of any of [9] to [11], or a plant comprising said cell.

### [Advantageous Effects of Invention]

The present invention can efficiently transform an organelle in a plant cell, especially a monocotyledon cell or a Brassicaceae plant cell, etc., and manufacture an organelle transformant by said method.

### [Brief Description of Drawings]

[Figure 1] Figure **1** is a schematic diagram of the screening method according to the present invention. The method using many non-Brassicaceae plant cells such as asterids (right) is a conventional method. In the drawing, ScACC1 indicates ACC1 derived from a budding yeast.
[Figure 2] Figure **2** is a schematic diagram of a nucleoid homogenization technology through gene drive.
[Figure 3] Figure **3** is a schematic diagram of a lipid synthesis pathway targeted by the present invention.
[Figure 4-1] Figure **4-1** is a diagram of the construct of pACC. The construct comprises only an ScACC1 gene of a herbicide selection marker.
[Figure 4-2] Figure **4-2** is a diagram of the construct of pACC-GD. The construct comprises ScACC1 of a herbicide selection marker and a gene cluster of Os.nCas9 of gene drive.
[Figure 4-3] Figure **4-3** is a diagram of the construct of pNC. The construct does not comprise ScACC1 or gene cluster of Os.nCas9.
[Figure 5] Figure **5** shows herbicide selected individuals that were redifferentiated after 6 weeks from the start of selection. (A) pNC (herbicide sensitive). (B) pACC (herbicide resistant).
[Figure 6] Figure **6** shows the effect of the herbicide concentration on redifferentiation for a callus introduced with plasmid pNC, pACC, or pACC-GD. (A) The number of individual Oryza sativa chloroplast transformants redifferentiated from a tralkoxydim containing redifferentiation selection medium at each concentration. (B) The number of individual Oryza sativa chloroplast transformants redifferentiated from a haloxyfop containing redifferentiation selection medium at each concentration.

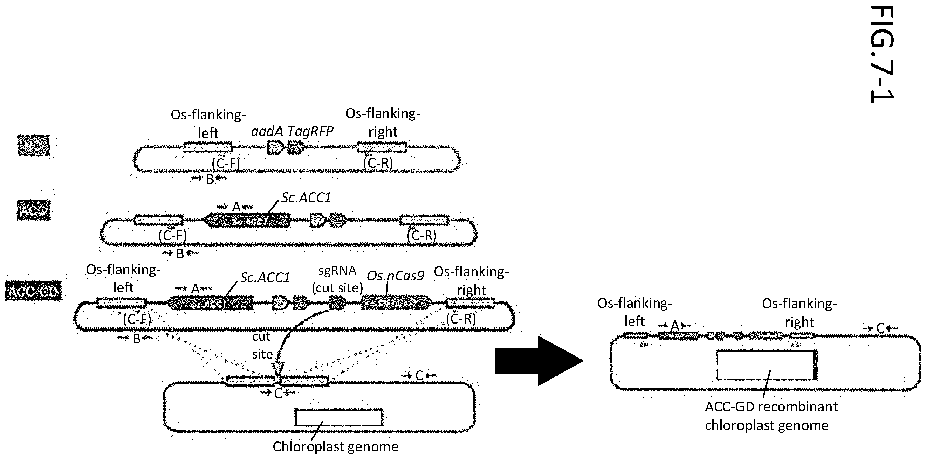

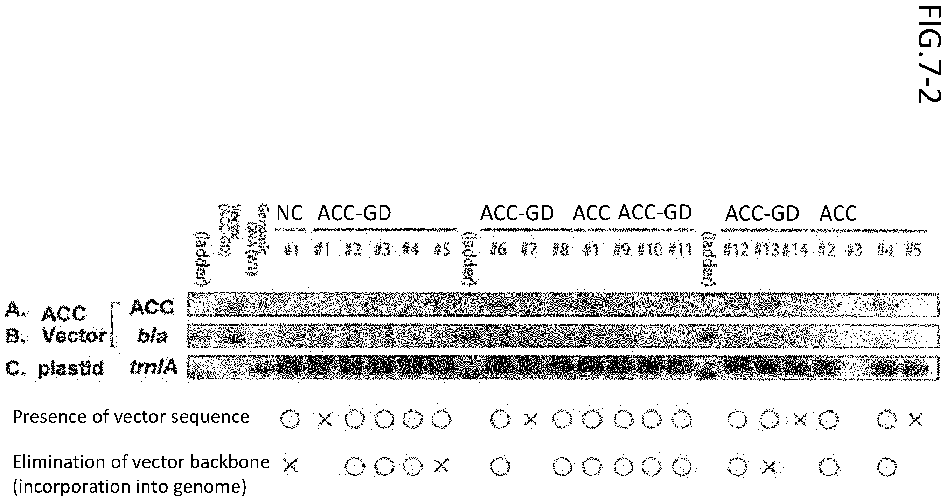

[Figure 7] Figure **7** shows detection of an Oryza sativa chloroplast transformant. (Top diagram) Introduced pNC, pACC, or pACC-GD plasmid and expected model diagram when the plasmid is inserted into the Oryza sativa chloroplast by homologous recombination. (Center diagram) The genome of a herbicide selected individual was used as a template for investigation by PCR. Primers targeting region A, B, or C in the (Top diagram) as an amplification region was used for amplification to check the presence/absence of a PCR fragment by agarose gel electrophoresis. (Bottom diagram) o indicates that a vector sequence was detected and × indicates that a vector sequence was not detected for each sample from the results of PCR. o indicates that incorporation into the genome was observed, and × indicates that incorporation into the genome was not observed.
[Figure 8] Figure **8** shows observation of Oryza sativa chloroplast genome transformants under a confocal microscope. (Left) Fluorescence image of TagRFP. (Right) Schematic diagram of the state of fluorescence of TagRFP of chloroplasts within cells.
[Figure 9-1] Figure **9-1** shows the effect of a combination of a herbicide and an antibiotic on Arabidopsis thaliana cultured cells. (A) A concentration gradient model for a combination of a herbicide and spectinomycin. (B) A concerted sensitivity test using pinoxaden and spectinomycin in 15 days of culture. (C) A concerted sensitivity test using cyhalofop butyl and spectinomycin in 15 days of culture.
[Figure 9-2] Figure **9-2** shows the effect of a combination of a herbicide and an antibiotic on Arabidopsis thaliana cultured cells. (D) A concerted sensitivity test using haloxyfop and spectinomycin in 15 days of culture. (E) A concerted sensitivity test using tepraloxydim and spectinomycin in 15 days of culture. (F) A concerted sensitivity test using tralkoxydim and spectinomycin in 15 days of culture.
[Figure 10] Figure **10** shows the configuration of an introduced construct. (A) pKK4. (B) pKK5.
[Figure 11] Figure **11** is a schematic diagram of the structure of a PGP1 gene and a PCR primer used for the detection. The placement of a PGP1 gene on the genome, and the configuration of the T-DNA insertion position in a pgp1-2 mutant, normal cDNA, and introduced DNA are shown. The functional site in a protein sequence encoded by the cDNA is shown at the top portion. The arrows in the diagram indicate the positions of a primer used in PCR for checking a transformed plant.
[Figure 12] Figure **12** shows complement of phenotype and transient expression of a gene in a chloroplast using pKK4. pgp1-2 mutants were grown in a sugar-containing medium. Buds on day 7 after budding were used and pKK4 was introduced by particle gun method, then the phenotype after culture for 13 days in a sugar-free medium was observed in treated sections with/without introduction of DNA. As a result, a phenotype that changes to yellow 100% was exhibited as shown in the right diagram when DNA was not introduced, while about 100 turned green in a pKK4 introduced line.

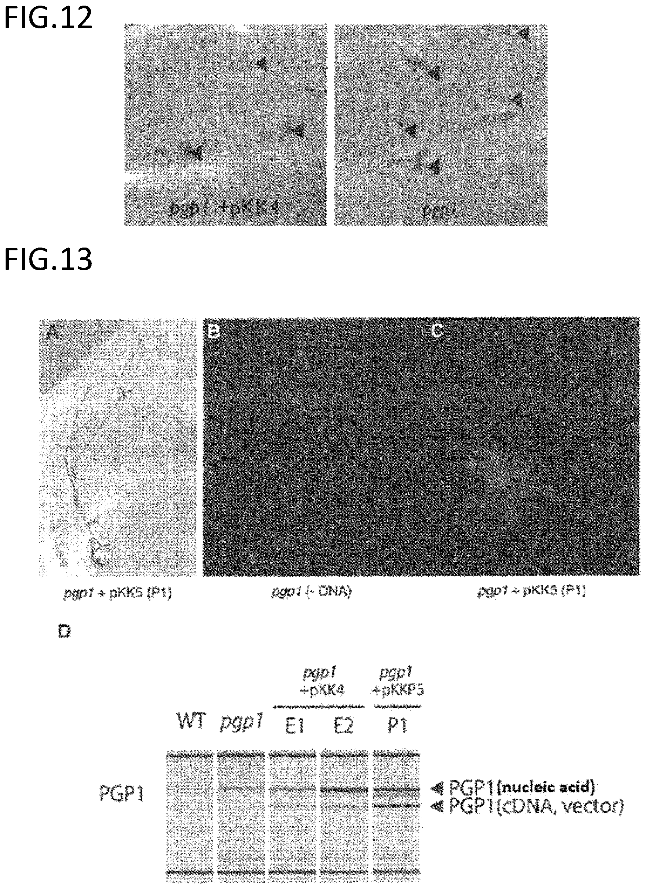

[Figure 13] Figure **13** shows creation of a chloroplast transformant using pKK5. (A) A chloroplast transformant (line number P1) obtained by introducing pKK5 through lipofection using pgp1-2 as a parent strain after 3 months of growing. The transformant did not turn yellow like pgp1-2 mutants, but turned green. A flower was also formed. (B to C) Observation of fluorescence within a cell by a microscope (B) pgp1-2 mutants have strong yellow autofluorescence, but not reddish fluorescence. (C) Chloroplasts of some of the cells in chloroplast transformant P1 were confirmed to emit fluorescence believed to be from TagRFP. (D) The introduced gene in the original mutant and the obtained transformant was checked through PCR. Amplification of the introduced PGP1 cDNA gene was clearly confirmed in P1.

### [Description of Embodiments]

### 1. Method of screening for monocotyledon cells with a transformed organelle

The present invention provides a method of screening for monocotyledon cells with a transformed organelle (hereinafter, also referred to as the "screening method of the invention (for monocotyledon)). The screening method of the invention (for monocotyledon) comprises the step of contacting an inhibitor of ACC2 (hereinafter, also referred to as the "ACC2 inhibitor") with a cell population comprising monocotyledon cells with a nucleic acid (hereinafter, also referred to as the "exogenous nucleic acid (for monocotyledon)"), which imparts resistance to the ACC2 inhibitor to the plant cells and enables expression of a protein of interest, introduced into an organelle. With this step, only cells with a successfully transformed organelle can survive with resistance to an ACC2 inhibitor, so that it is possible to screen for only cells with a transformed organelle. Another embodiment provides a method of manufacturing a cell population having purified monocotyledon cells with a transformed organelle, comprising the step of contacting an ACC2 inhibitor with a cell population comprising monocotyledon cells with an exogenous nucleic acid (for monocotyledon) introduced into an organelle (hereinafter, also referred to as the "manufacturing method of the invention (for monocotyledon)". An ACC2 inhibitor may be an inhibitor that specifically inhibits only ACC2 or concurrently inhibits another enzyme such as ACC1.

An exogenous nucleic acid (for monocotyledon) comprises a gene encoding acetyl-CoA carboxylase having resistance to an ACC2 inhibitor (hereinafter, also referred to as the "ACC2 inhibitor resistant gene") and a gene encoding a protein of interest (hereinafter, also referred to as the "protein of interest encoding gene"). The ACC2 inhibitor resistant gene and the protein of interest encoding gene described above may be present on a single nucleic acid or on different nucleic acids. Thus, an exogenous nucleic acid (for monocotyledon) may consist of only one or a plurality of nucleic acids.

As used herein, "organelle" refers to an organelle having a DNA other than a nuclear DNA. Examples thereof include plastids and mitochondria. Examples of the plastids include chloroplast, etioplast, amyloplast, elaioplast, chromoplast, and leucoplast, but chloroplast is particularly preferable.

A monocotyledon from which the cells used in the present invention are derived is not particularly limited, as long as it has an ACC2 gene. Examples thereof include plants of the family Poaceae, plants of the family Amaryllidaceae, etc. Examples of the plants of the family Poaceae include plants of the genus Oryza, Triticum, Hordeum, Secale, Saccharum, Sorghum, and Zea. Specific examples include, but are not limited to, Zea mays, Sorghum bicolor, Triticum aestivum, Oryza sativa, Avena sativa, Hordeum vulgare, Secale cereale, and Setaria italica. Preferred plants of the family Poaceae are Zea mays, Triticum aestivum, and Oryza sativa. Examples of the plants of the family Amaryllidaceae include, but are not limited to, Allium cepa, Allium fistulosum, and Allium sativum. Preferred plants of the family Amaryllidaceae are Allium cepa and Allium fistulosum. "Plant cells" herein include cultured cells as well as cells in a plant. Plant cells also include plant cells in various forms such as suspension culture cells, protoplasts, sections of a leaf, sections of a root, calluses, immature embryos, pollens, etc.

As used herein, ACC2 is one subtype of acetyl-CoA carboxylase, referring to an enzyme that catalyzes a reaction for generating malonyl CoA (hereinafter, also referred to as "malonyl CoA generation reaction") by using acetyl-CoA and hydrogen carbonate as the substrates. Acetyl-CoA carboxylase may be either a complex consisting of biotin carboxylase (BC), biotin carboxyl carrier protein (BCCP), carboxyl transferase (CTα, CTβ) (hereinafter, also referred to as the "prokaryotic enzyme") or a protein with functions of each protein constituting the complex that are present in one protein as domains (hereinafter, also referred to as the "eukaryotic enzyme"). Among proteins constituting a prokaryotic enzyme that is a complex, BCCP is encoded by a CAC1 gene, BC is encoded by a CAC2 gene, CTα is encoded by a CAC3 gene, and CTβ is encoded by an AccD gene (these proteins are also referred to as CAC1, CAC2, CAC3, and AccD herein, respectively). Examples of eukaryotic enzymes include ACC2 as well as ACC1. Among the genes described above, the AccD gene is present in a chloroplast genome, and the CAC1 gene, CAC2 gene, CAC3 gene, and ACC2 gene are present in the nuclear genome in a plant cell. Proteins encoded by the genes described above that are present in the nuclear genome are translated in the cytoplasm, and then migrate to a chloroplast. The ACC1 gene is present in the nuclear genome, and a protein encoded by said gene remains in the cytoplasm after translation in the cytoplasm. A gene encoding a prokaryotic enzyme is absent in monocotyledons, whereas a gene encoding a eukaryotic enzyme of the type that localizes in a chloroplast is absent in many dicotyledons other than Brassicaceae (e.g., asterids, etc.). The majority of plants of the family Brassicaceae, etc. has both a prokaryotic enzyme and a eukaryotic enzyme in a chloroplast.

ACC2 inhibitors used in the screening method or manufacturing method of the invention (for monocotyledon) are not particularly limited, as long as a reaction of ACC2 described above can be inhibited. Examples thereof include compounds with an HRAC (Herbicide Resistance Action Committee) classification of A. Examples of such ACC2 inhibitors include allyloxypropionate compounds, cyclohexanedione compounds, phenylpyrazoline (DEN, pinoxaden) compounds, etc. More specific examples include alloxydim (primary applied plants: annual plants of the family Poaceae), butroxydim (primary applied plants: annual plants of the family Poaceae), chlorazifop (primary applied plants: plants of the family Poaceae), clethodim (primary applied plants: plants of the family Poaceae), clodinafop (primary applied plants: annual plants of the family Poaceae), clofop (primary applied plants: annual plants of the family Poaceae), cloproxydim (primary applied plants: plants of the family Poaceae), cycloxydim (primary applied plants: annual plants of the family Poaceae), cyhalofop butyl (primary applied plants: plants of the family Poaceae), diclofop (primary applied plants: Avena fatua, Lolium multiflorum), fenoxaprop ethyl (primary applied plants: annual plants of the family Poaceae), fenoxaprop-P (primary applied plants: annual plants of the family Poaceae), fluazifop (primary applied plants: plants of the family Poaceae), fluazifop P (primary applied plants: plants of the family Poaceae), haloxyfop (primary applied plants: plants of the family Poaceae), haloxyfop P (primary applied plants: annual and perennial plants of the family Poaceae), isoxapyrifop (primary applied plants: annual plants of the family Poaceae), metamifop (primary applied plants: annual plants of the family Poaceae), pinoxaden (primary applied plants: plants of the family Poaceae), profoxydim (primary applied plants: plants of the family Poaceae), propaquizafop (primary applied plants: plants of the family Poaceae), quizalofop ethyl (primary applied plants: weed of the family Poaceae), quizalofop P ethyl (primary applied plants: plants of the family Poaceae), sethoxydim (primary applied plants: plants of the family Poaceae), tepraloxydim (primary applied plants: plants of the family Poaceae), tralkoxydim (primary applied plants: annual weeds of the family Poaceae), etc. In particular, pinoxaden, haloxyfop, tepraloxydim, or tralkoxydim is preferred.

Plant cells or a cell population comprising said cells may be contacted with an ACC2 inhibitor by adding the ACC2 inhibitor to a medium comprising the cells or cell population, or by seeding the cells or cell population in a medium supplemented with the ACC2 inhibitor in advance.

The concentration of an ACC2 inhibitor can be appropriately determined depending on the type of ACC2 inhibitor. For example, the concentration of tralkoxydim in a medium is preferably 0.5 to 2 µM, and the concentration of haloxyfop in a medium is preferably 0.1 to 0.5 µM, but the concentrations are not limited to these ranges.

Acetyl-CoA carboxylase having resistance to an ACC2 inhibitor, which is used in the screening method or manufacturing method of the invention (for monocotyledon), can be appropriately selected in accordance with the type of ACC2 inhibitor. For example, when an ACC2 inhibitor is used in cells of the corresponding primary applied plant, acetyl-CoA carboxylase derived from a plant other than the primary applied plant may be used, or resistance to the inhibitor may be imparted by introducing a mutation to acetyl-CoA derived from the primary applied plant at a site binding to the inhibitor, etc. Further, a eukaryotic enzyme may be used, or a prokaryotic enzyme may be used for the acetyl-CoA carboxylase described above. Thus, in one embodiment of the invention, acetyl-CoA carboxylase derived from a microorganism (e.g., Escherichia bacteria, Bacillus bacteria, budding yeast, fission yeast, etc.), a mammal (e.g., human, mouse, pig, cow, horse, monkey, etc.), etc. can be used as acetyl-CoA carboxylase to an ACC2 inhibitor. More specifically, a complex consisting of CAC1, CAC2, CAC3, and AccD, ACC1, ACC2 derived from an organism other than a monocotyledon (more preferably other than plants), etc. can be used. Since the majority of ACC2 inhibitors do not act on prokaryotic enzymes, use of a prokaryotic enzyme is preferred. The acetyl-CoA carboxylase described above may be a functional fragment thereof that can catalyze a malonyl-CoA generation reaction. As used herein, "acetyl-CoA carboxylase" also encompasses the functional fragments thereof. More specific examples of the acetyl-CoA carboxylase described above include ACC1 derived from a budding yeast (SEQ ID NO: 2 (nucleotide sequence encoding ACC1 is set forth in SEQ ID NO: 1)), proteins consisting of the amino acid sequence set forth in SEQ ID NO: 2 with 1 or several (e.g., 2, 3, 4, 5, 6, 7, 8, 9, or 10) amino acid deletions, substitutions, insertions, and/or additions, proteins consisting of an amino acid sequence with about 90% or greater, preferably about 95% or greater, more preferably about 97% or greater, particularly preferably about 98% or greater, and most preferably about 99% or greater identity to the amino acid sequence set forth in SEQ ID NO: 1, etc.

2. Method of screening for Brassicaceae plant cells, etc.

### with a transformed organelle

In another embodiment, the present invention provides a method of screening for Brassicaceae plant cells with a transformed organelle (hereinafter, also referred to as the "screening method of the invention (for plants of the family Brassicaceae, etc.)"). The screening method of the invention (for plants of the family Brassicaceae, etc.) comprises the step of contacting an ACC2 inhibitor, spectinomycin, and a cell population comprising Brassicaceae plant cells with a nucleic acid, which imparts resistance to the ACC2 inhibitor or spectinomycin to the plant cells and enables expression of a protein of interest (hereinafter, also referred to as the "exogenous nucleic acid (for plants of the family Brassicaceae, etc.)") introduced into an organelle. With this step, only cells with a successfully transformed organelle can survive with resistance to an ACC2 inhibitor or spectinomycin, so that it is possible to screen for only cells with a transformed organelle. Another embodiment provides a method of manufacturing a cell population with purified Brassicaceae plant cells with a transformed organelle, comprising the step of contacting a gene resistant to an ACC2 inhibitor or spectinomycin with a cell population comprising Brassicaceae plant cells with an exogenous nucleic acid (for plants of the family Brassicaceae, etc.) introduced into an organelle (hereinafter, also referred to as the "manufacturing method of the invention (for plants of the family Brassicaceae, etc.)".

As used herein, a cell population with purified cells refers to a cell population with an increased ratio of the cells accounted for in a cell population compared to a cell population prior to contact with an ACC2 inhibitor.

An exogenous nucleic acid (for plants of the family Brassicaceae, etc.) comprises a gene resistant to an ACC2 inhibitor or a gene resistant to spectinomycin (hereinafter, also referred to as the "spectinomycin resistant gene") and a protein of interest encoding gene. The ACC2 inhibitor resistant gene or spectinomycin resistant gene and the protein of interest encoding gene described above may be present on a single nucleic acid or on different nucleic acids. Thus, an exogenous nucleic acid (for plants of the family Brassicaceae, etc.) may consist of only one or a plurality of nucleic acids.

Plants from which cells used in the present invention are derived are not particularly limited, as long as a eukaryotic enzyme migrates to a chloroplast. Examples thereof include Arabidopsis lyrata of the genus Arabidopsis, Camelina rubella and Camelina sativa of the genus Camelina, Brassica oleracea, Brassica napus, and Brassica rapa of the genus Brassica, and other Brassicaceae plants. Accordingly, in one embodiment of the invention, Brassicaceae plant cells can be used. For example, whether acetyl-CoA carboxylase migrates to a chloroplast after translation in plants without findings regarding migration to a chloroplast can be studied by testing localization through fluorescence by using, for example, a protein with a fluorescent protein fused to an end of the enzyme, etc.

Examples of ACC2 inhibitors used in the screening method or manufacturing method of the invention (for plants of the family Brassicaceae, etc.) include the same inhibitors mentioned in 1. Examples of genes resistant to an ACC2 inhibitor include genes encoding acetyl-CoA carboxylase having resistance to an ACC2 inhibitor described in 1. Such genes can be appropriately selected in accordance with the type of ACC2 inhibitor. For example, when an ACC2 inhibitor is used in cells of the corresponding primary applied plant, acetyl-CoA carboxylase derived from a plant other than the primary applied plant may be used, or resistance to the inhibitor may be imparted by introducing a mutation to acetyl-CoA derived from the primary applied plant or others at a site binding to the inhibitor, etc. Further, a eukaryotic enzyme may be used, or a prokaryotic enzyme may be used for the acetyl-CoA carboxylase described above. Thus, in one embodiment of the invention, acetyl-CoA carboxylase derived from a microorganism (e.g., Escherichia bacteria, Bacillus bacteria, budding yeast, fission yeast, etc.), a mammal (e.g., human, mouse, pig, cow, horse, monkey, etc.), etc. can be used as acetyl-CoA carboxylase to an ACC2 inhibitor. More specifically, a complex consisting of CAC1, CAC2, CAC3, and AccD, ACC1, ACC2 derived from an organism other than plants of the family Brassicaceae (more preferably other than plants), etc. can be used. Since the majority of ACC2 inhibitors do not act on prokaryotic enzymes, use of a prokaryotic enzyme is preferred. The acetyl-CoA carboxylase described above may be a functional fragment thereof that can catalyze a malonyl-CoA generation reaction. As used herein, "acetyl-CoA carboxylase" encompasses the functional fragments thereof. More specific examples of the acetyl-CoA carboxylase described above include ACC1 derived from a budding yeast (SEQ ID NO: 2), proteins consisting of the amino acid sequence set forth in SEQ ID NO: 2 with 1 or several (e.g., 2, 3, 4, 5, 6, 7, 8, 9, or 10) amino acid deletions, substitutions, insertions, and/or additions, proteins consisting of an amino acid sequence with about 90% or greater, preferably about 95% or greater, more preferably about 97% or greater, particularly preferably about 98% or greater, and most preferably about 99% or greater identity to the amino acid sequence set forth in SEQ ID NO: 1, etc.

Instead of a gene resistant to an ACC2 inhibitor, a gene resistant to spectinomycin may be used. Specific examples thereof include, but are not limited to, the gene (SEQ ID NO: 3) encoding aminoglycoside adenine transferase (aadA) (SEQ ID NO: 4), genes encoding a protein consisting of the amino acid sequence set forth in SEQ ID NO: 4 with 1 or several (e.g., 2, 3, 4, 5, 6, 7, 8, 9, or 10) amino acid deletions, substitutions, insertions, and/or additions, genes encoding a protein consisting of an amino acid sequence with about 90% or greater, preferably about 95% or greater, more preferably about 97% or greater, particularly preferably about 98% or greater, and most preferably about 99% or greater identity to the amino acid sequence set forth in SEQ ID NO: 4, etc. Identity of an amino acid sequence herein can be calculated under the following conditions (expected value = 10; allow gap; matrix = BLOSUM62; filtering = OFF) by using a homology calculation algorithm NCBI BLAST (National Center for Biotechnology Information Basic Local Alignment Search Tool).

Plant cells or a cell population comprising said cells may be contacted with an ACC2 inhibitor and spectinomycin by adding the ACC2 inhibitor and/or spectinomycin to a medium comprising the cells or cell population, or by seeding the cells or cell population in a medium supplemented with the ACC2 inhibitor and/or spectinomycin in advance. An ACC2 inhibitor and spectinomycin may be contacted with plant cells or cell population comprising said cells at the same timing, or contacted at different times.

The concentration of an ACC2 inhibitor can be appropriately determined depending on the type of ACC2 inhibitor. For example, the concentration of pinoxaden in a medium is preferably 0.1 to 10 µM, the concentration of haloxyfop in a medium is preferably 1 to 10 µM, the concentration of tepraloxydim in a medium is preferably 10 to 50 µM, and the concentration of tralkoxydim in a medium is preferably 10 to 50 µM, but the concentrations are not limited to these ranges.

### 3. Method of manufacturing a plant cell with a transformed organelle

The present invention provides a method of manufacturing a plant cell with a transformed organelle (hereinafter, also referred to as the "manufacturing method of the invention (for an enzymatic function attenuated plant)"). The manufacturing method of the invention (for an enzymatic function attenuated plant) comprises the step of introducing a nucleic acid, which enables expression of a protein that compensates for a function of an enzyme involved with a synthesis pathway of an essential lipid-soluble compound in an organelle and expression of a protein of interest (hereinafter, also referred to as the "exogenous nucleic acid (for an enzymatic function attenuated plant)" into an organelle of a plant cell with a function of the enzyme attenuated (attenuation of a function also encompasses loss of function herein). With this step, only cells with a successfully transformed organelle can form a normal organelle by recovery of a synthesis pathway of an essential lipid-soluble compound, so that it is possible to screen for only cells with a transformed organelle using formation of a normal organelle as an indicator. Thus, another embodiment provides a method of screening for plant cells with a transformed organelle, comprising the step of introducing an exogenous nucleic acid (for an enzymatic function attenuated plant) into an organelle of a plant cell with an attenuated function of an enzyme involved with a synthesis pathway of an essential lipid-soluble compound (hereinafter, also referred to as the "screening method of the invention (for an enzymatic function attenuated plant)"). Examples of the enzymes with an attenuated function described above include, but are not limited to, loss-of-function mutants, mutants without the enzyme, etc. An essential lipid-soluble compound may be a compound other than a glycerolipid. The compound is not particularly limited, as long as it is an essential compound with a lipid-soluble property such as sterols, quinones, sphingolipids, porphyrins, and membrane localized or transmembrane proteins (e.g., TIC-TOC/TIM-TOM complex).

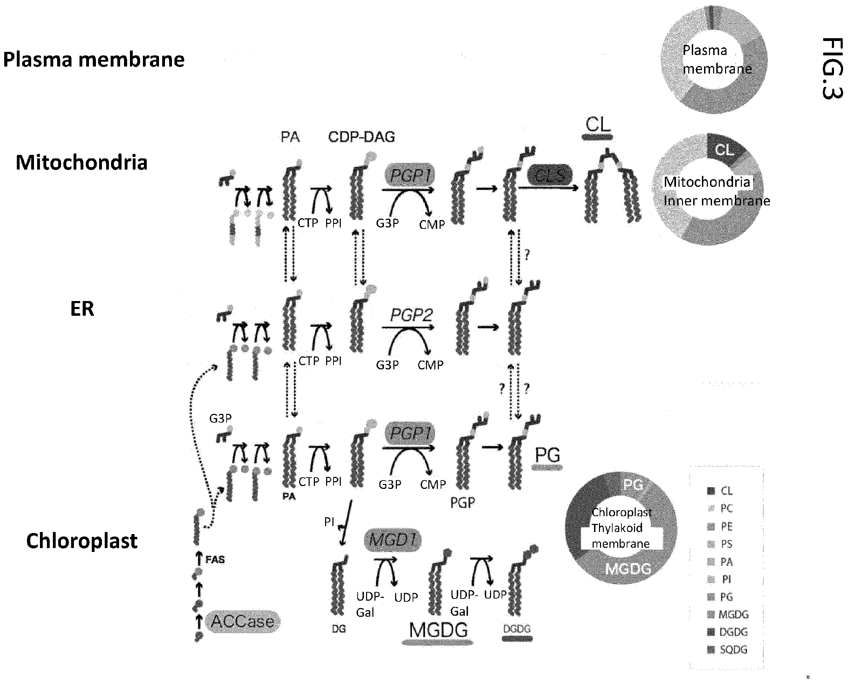

Figure **3** shows a schematic diagram of a glycerolipid synthesis pathway among lipid-soluble compound synthesis pathways. A glycerolipid synthesis pathway may also be simply referred to as "lipid synthesis pathway" herein. Enzymes involved with said pathway may be collectively referred to as the "group of enzymes involved with a lipid synthesis pathway". Examples of the enzymes involved with a lipid synthesis pathway described above include, but are not limited to, glycerol-3-phosphate acyltransferase (e.g., GPAT) that catalyzes synthesis from glycerol-3-phosphate (G3P) to lysophosphatidic acid (LPA), lysophosphatidic acid acyltransferase (LPAT) that catalyzes synthesis from LPA to phosphatidic acid (PA), CDP-diacylglycerol synthase (e.g., Cds1 and Tam41) that catalyzes synthesis from PA to CDP-diacylglycerol (CDP-DAG), phosphatidylglycerolphosphate synthase (e.g., PGP1) that catalyzes synthesis from CDP-DAG to phosphatidylglycerophosphate (PGP), phosphatidylglycerol synthase (phosphatase) (e.g., PGPP) that catalyzes synthesis of PGP (dephosphorylation of PGP) into phosphatidylglycerol (PG), cardiolipin synthase (e.g., CLS) that catalyzes synthesis from PG to cardiolipin (CL), monogalactosyldiacylglycerol synthase (e.g., MGD1) that catalyzes synthesis from diacylglycerol (DG) to monogalactosyldiacylglycerol (MGDG), digalactosyldiacylglycerol synthase (e.g., DGD1 and DGD2) that catalyzes synthesis from MGDG to digalactosyldiacylglycerol (DGDG), etc. In particular, PGP1, CLS, or MGD1 is preferred.

As plant cells having a loss-of-function mutation in an enzyme involved with an essential lipid-soluble compound synthesis pathway described above or plant cells without a deletion the enzyme (these plant cells with inactivated enzymatic activity or deletion of the enzyme are also referred to as mutants), a known mutant may be used, or such mutants may be newly prepared. Examples of known mutants that have been reported include CL synthase mutants (Katayama, K. and Wada, H. (2012) Cytologia 77: 123-129), PG synthase mutants (Hagio, M. et al. (2002) Plant Cell Physiol. 43: 1456-64), MGDG synthase mutants (Kobayashi et al. (2007) PNAS 104: 17216-21), etc. The mutants described above can be introduced with a loss-of-function mutation by deleting or inserting a base from/into a nucleotide sequence encoding the enzyme by using genome editing, etc. or prepared by substituting a gene encoding the enzyme with another gene through knock-in, etc. The function of an enzyme may be attenuated by using an inhibitor of an enzyme involved with a lipid synthesis pathway instead of introducing a mutation into a gene. In such a case, a protein that compensates for the function of the enzyme having resistance to the inhibitor can be used. Examples of such inhibitors include, but are not limited to, MGD1 inhibitor Galvestine-1 and derivatives thereof (Botte, CY et al. (2011) Nature Chemical Biology 7: 834-842), etc.

An exogenous nucleic acid (for an enzymatic function attenuated plant) comprises a gene encoding a protein that compensates for the function of an enzyme involved with an essential lipid-soluble compound synthesis pathway described above (hereinafter, also referred to as the "compensating protein") (hereinafter, also referred to as the "compensating gene") and a protein of interest encoding gene. The compensating gene and protein of interest encoding gene described above may be present on a single nucleic acid or on different nucleic acids. Thus, an exogenous nucleic acid (for an enzymatic function attenuated plant) may consist of only one or a plurality of nucleic acids.

As the compensating protein described above, an enzyme with a normal function (e.g., having enzymatic activity) (e.g., wild-type enzyme) corresponding to a mutant enzyme involved with an essential lipid-soluble compound synthesis pathway can be typically used. A mutation with no or low effect on enzymatic activity may be introduced into such a compensating protein. If, for example, an organelle migration signal is added to a compensating protein, the signal can be deleted to prevent the formation of an essential lipid-soluble compound in an organelle even if an exogenous nucleic acid (for an enzymatic function attenuated plant) is introduced into a place other than an organelle. Examples of enzymes having resistance to an inhibitor of an enzyme involved with an essential lipid-soluble compound synthesis pathway include, but are not limited to, mutants prepared from introducing an appropriate mutation into a wild-type enzyme, enzymes derived from a different species having resistance to the inhibitor, etc.

Plant cells treated with the mutant or inhibitor described above targeted for an essential lipid-soluble compound in a chloroplast generally exhibits an albino phenotype due to dysplasia of chloroplasts. Meanwhile, when an organelle is transformed with an exogenous nucleic acid (for an enzymatic function attenuated plant), a green phenotype is exhibited. Thus, a transformant can be screened by the hue of a chloroplast, or transformation can be confirmed by a method that is known such as PCR.

Plant cells used in the manufacturing method or screening method of the invention (for an enzymatic function attenuated plant) are not particularly limited, as long as they are cells with an organelle. Plants from which the plant cells are derived are not particularly limited, but are preferably monocotyledons or dicotyledons. Examples of monocotyledons include, but are not limited to, plants of the family Poaceae and plants of the family Amaryllidaceae. Examples of the plants of the family Poaceae include plants of the genus Oryza, Triticum, Hordeum, Secale, Saccharum, Sorghum, and Zea. Specific examples thereof include, but are not limited to, Zea mays, Sorghum bicolor, Triticum aestivum, Oryza sativa, Avena sativa, Hordeum vulgare, Secale cereale, and Setaria italica. Preferred plants of the family Poaceae are Zea mays, Triticum aestivum, and Oryza sativa. Examples of the plants of the family Amaryllidaceae include, but are not limited to, Allium cepa, Allium fistulosum, and Allium sativum. Preferred plants of the family Amaryllidaceae are Allium cepa and Allium fistulosum.

Examples of dicotyledons include, but are not limited to, Brassicaceae plants, Fabaceae plants, Solanaceae plants, Cucurbitaceae plants, Convolvulaceae plants, and Asteraceae plants. Examples of Brassicaceae plants include plants of the genus Raphanus, Brassica, Arabidopsis, Wasabia, and Capsella. Specific examples thereof include, but are not limited to, Brassica rapa subsp. Pekinensis, Brassica rapa L. var. nippo-oleifera, Brassica oleracea L. var. capitate, Brassica oleracea var. botrytis, Raphanus sativus var. hortensis, Brassica napus subsp. Napus, Arabidopsis thaliana, Eutrema japonicum, and Capsella bursa-pastoris. Preferred Brassicaceae plants are Arabidopsis thaliana, Brassica rapa subsp. Pekinensis, and Brassica rapa L. var. nippo-oleifera. Examples of Fabaceae plants include, but are not limited to, Glycine max, Vigna angularis, Phaseolus vulgaris, and Vigna unguiculata. Preferred Fabaceae plants are Glycine max and Phaseolus vulgaris. Examples of Solanaceae plants include, but are not limited to, Nicotiana tabacum, Solanum lycopersicum, Petunia x hybrida, Solanum melongenam, and Solanum tuberosum. Preferred Solanaceae plants are Nicotiana tabacum, Solanum lycopersicum, and Petunia x hybrid. Examples of Cucurbitaceae plants include, but are not limited to, Cucumis melo var. makuwa, Cucumis sativus, Cucumis melo, and Citrullus lanatus. Preferred Cucurbitaceae plants are Cucumis melo var. makuwa. Examples of Asteraceae plants include, but are not limited to, Helianthus annuus, Chrysanthemum × morifolium, and Taraxacum officinale. Preferred Asteraceae plants are Helianthus annuus. Examples of Convolvulaceae plants include, but are not limited to, Ipomoea nil, Ipomoea batatas, and Calystegia japonica. Preferred Convolvulaceae plants are Ipomoea batatas.

Examples of plants other than those described above include plants of the family Rosaceae, Lamiaceae, Liliaceae, Chenopodiaceae, Polygonaceae, Amaranthaceae, Apiaceae, Araceae, etc. as well as any tree species, any fruit tree species, plants of the family Moraceae (e.g., rubber), and plants of the family Malvaceae (e.g., cotton).

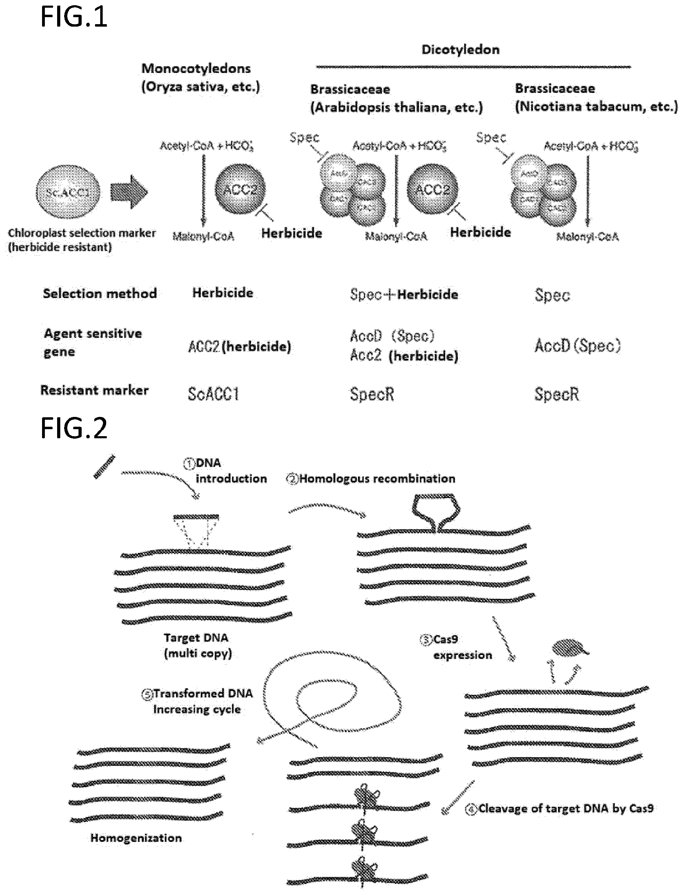

Hereinafter, methods encompassing each of the screening method of the invention (for monocotyledons), the screening method of the invention (for plants of the family Brassicaceae, etc.), and the screening method of the invention (for an enzymatic function attenuated plant) may be simply referred to as the "screening method of the invention". Likewise, methods encompassing each of the manufacturing method of the invention (for monocotyledons), the manufacturing method of the invention (for plants of the family Brassicaceae, etc.), and the manufacturing method of the invention (for an enzymatic function attenuated plant) may be simply referred to as the "manufacturing method of the invention". The outline of the screening method of the invention is shown as Figure 1. Nucleic acids encompassing each of the exogenous nucleic acid (for monocotyledons), the exogenous nucleic acid (for plants of the family Brassicaceae, etc.), and the exogenous nucleic acid (for an enzymatic function attenuated plant) may be simply referred to as the "exogenous nucleic acid".

Each gene contained in an exogenous nucleic acid is also referred to as the "exogenous gene" herein. As used herein, "cell with a transformed organelle" refers to a cell in which at least one organelle therein is transformed. "Transformed organelle" means that at least one nucleoid in the organelle is transformed (e.g., an exogenous gene is incorporated into a nucleoid, and a protein encoded by the gene is expressed).

Even toxic proteins can accumulate in the cytoplasm within the organelle. Thus, examples of a protein of interest encoding gene used in the screening method of the invention or the manufacturing method of the invention include, but are not particularly limited to, merA genes, pest resistant genes, herbicide resistant genes, genes associated with biosynthesis of carotenoid, vitamin E, etc. having antioxidant action, and fructose 1,6-bisphosphatase/sedoheptulose 1,7-bisphosphatage genes involved with biosynthesis, etc. If an exogenous nucleic acid has a metallothionein gene such as merA gene in an organelle, a cell introduced with said vector can acquire resistance to toxic heavy metal or mercury in the environment such as the soil. If an exogenous nucleic acid has a pest resistant gene or antibiotic resistant gene, a cell introduced with said exogenous nucleic acid can acquire pest resistance or resistance to infectious bacteria. If an exogenous nucleic acid has a gene associated with biosynthesis of carotenoid, vitamin E, etc. having antioxidant action, a cell introduced with said nucleic acid can have improved content of a useful component such as carotenoid or vitamin E in oil and fat generated by the cell. If an exogenous nucleic acid has a blue-green algae derived fructose 1,6-bisphosphatase/sedoheptulose 1,7-bisphosphatage gene involved with biosynthesis, etc. a cell introduced with the nucleic acid can be taller, have a greater area of leaves, growth faster, and have an enhanced ability to synthesize sugar or starch compared to wild-type.

If a toxic protein (CMS causing protein, etc.) is expressed within an organelle, development of pollen is inhibited to result in cytoplasmic male sterility (CMS). Meanwhile, since fertilization is possible if pollen from another pollen parent is provided, this phenomenon is utilized in the production of seeds of an F1 hybrid, etc. The protein of interest encoding gene described above is not particularly limited. Examples thereof include orf79 gene, orfH79 gene, orf284 gene, orf352 gene, urf13 gene, orf355 gene, atp6-C gene, orf256 gene, orf260 gene, orf138 gene, orf125 gene, orf224 gene, orf222 gene, orf463 gene, orfH522 gene, orf107 gene, preSatp6 gene, orf129 gene, G cox2 gene, pcf gene, orf239 gene, Ψatp6-2 gene, orf456 gene, other CMS causing genes, etc. If an exogenous nucleic acid has a CMS causing gene, etc., an individual introduced with the nucleic acid is inhibited from developing germ cells and does not self-fertilize. Thus, the individual is readily cross-pollinated and can form an F1 seed. If an individual with a suitable fertility recovering factor is used, an individual still having a gene of interest in an organelle can also be maintained.

Even ribonucleic acids which are subjected to a host specific unique metabolism in the cytoplasm can accumulate while remaining in a transcribed form within an organelle. Thus, examples of the protein of interest encoding gene include, but are not particularly limited to, constructs that allow expression of target pest specific conversed sequence contained in a gene essential to a pest such as the pest's acetylcholinesterase gene, chitin synthase gene, or actin gene, resulting in a double stranded ribonucleic acid, etc. If an exogenous nucleic acid comprises a construct that allows expression of a pest specific conversed region to be a double stranded ribonucleic acid, double stranded ribonucleic acid that is not plant specifically metabolized is metabolized uniquely within the pest to produce RNAi and expression of an essential gene is suppressed, so that fatality of a pest that has consumed a cell introduced with said nucleic acid or an effect of aversion to the cell can be exhibited.

### 4. Method of increasing the ratio of nucleoids or organelles having an introduced gene

Since an organelle that is not transformed in plant cells manufactured by the manufacturing method of the invention or plant cells screened out by the screening method of the invention is destroyed without forming a lipid bilayer by an ACC2 inhibitor, only organelles that are transformed grow by culturing the plant cells, so that the ratio of organelles within a cell can be increased.

The screening method or manufacturing method of the invention can comprise the step of increasing a ratio of transformed nucleoids to nucleoids contained in an organelle by destroying nucleoids other than nucleoids transformed with an exogenous nucleic acid and having an exogenous gene and selectively replicating nucleoids having the gene, prior to contacting an ACC2 inhibitor with plant cells. For example, the CRISPR-Cas system can be used to perform such a step. Specifically, a gene encoding a Cas effector protein and a gene encoding a guide RNA are included in an exogenous nucleic acid, and the guide RNA is targeted to a sequence, which is included in a nucleotide but is eliminated in a nucleoid with an exogenous nucleic acid incorporated therein by homologous recombination, so that nucleoids without the exogenous nucleic acid can be selectively destroyed, and continuation of culture of cells can increase the ratio of nucleoids with an introduced exogenous gene to nucleoids contained in an organelle. This method is referred to as a nucleoid homogenization technology through gene drive. A schematic diagram of this method is shown as Figure **2****.**

A Cas effector protein used in the nucleoid homogenization technology described above is not particularly limited, as long as it is an effector protein belonging to a CRISPR system, which can form a complex with a guide RNA and recognize and bind to a target nucleotide sequence in a gene or interest and a protospacer adjacent motif (PAM) adjacent thereto, but is preferably Cas9 or Cpf1. Examples of Cas9 include, but are not limited to, Streptococcus pyogenes derived Cas 9 (SpCas9; PAM sequence (5'→3' direction; the same applies hereinafter) NGG (N is A, G, T, or C; the same applies hereinafter)), Streptococcus thermophilus derived Cas9 (StCas9; PAM sequence NNAGAAW), Neisseria meningitidis derived Cas9 (MmCas9; PAM sequence NNNNGATT), etc. Preferably, Cas9 is SpCas9 which has few restriction due to PAM (PAM sequence is substantially 2 bases, so theoretically almost anywhere on the genome can be targeted). Examples of Cpf1 include, but are not limited to, Francisella novicida derived Cpf1 (FnCpf1; PAM sequence TTN), Acidaminococcus sp. derived Cpf1 (AsCpf1; PAM sequence TTTN), Lachnospiraceae bacterium derived Cpf1 (LbCpf1; PAM sequence TTTN), etc.

The Cas effector proteins described above may have nickase activity, with only the cleaving capability of one of the strands of a target double stranded DNA inactivated. For example for SpCas9, a D10A mutant lacking the cleaving capability of a strand opposite of a strand forming a complementary strand with a guide RNA with the 10^{th} Asp residue converted to an Ala residue (thus having nickase activity on a strand forming a complementary strand with a guide RNA), or an H840A mutant lacking the cleaving capability of a strand forming a complementary strand with a guide RNA with the 840^{th} His residue converted into an Ala residue (thus having nickase activity on a strand opposite of a strand forming a complementary strand with a guide RNA) can be used.

Plant cells can be cultured in accordance with a known method, depending on the type. As a medium used for culturing, a solid medium (e.g., agar medium, agarose medium, gellan gum medium, etc.) is preferred. A medium preferably contains a carbon source, nitrogen source, inorganic matter, etc. that are required for the growth of a transformant. For example, an N6 medium, MS medium, LS medium, B5 medium, etc. is used as a basal medium. A plant growth substance (e.g., auxins, cytokinins, etc.), etc. may be added when appropriate to a medium. The pH of a medium is preferably about 5 to about 8. Generally, the culture temperature can be appropriately selected within the range of about 20°C to about 35°C depending on the type of plant cells. For example, Oryza sativa cells can be generally cultured at 28 to 33°C and preferably at 30 to 33°C.

The medium described above may also contain sugars as a carbon source, vitamins, support to solidify the medium, etc. Examples of sugars include glucose, sucrose, etc. The amount of sugar added is about 1 to 10% by weight and preferably about 2 to 5% by weight. Examples of vitamins include thiamine hydrochloride, pyridoxine hydrochloride, nicotinic acid, inositol, etc. Examples of support include agar, gellan gum, paper bridge, etc. The medium described above may contain amino acid (e.g., glycine, etc.), adenine, coconut water, etc.

### 5. Cell having an organelle transformed by a vector of the invention

The present invention also provides a plant cell obtained by the screening method or manufacturing method of the invention, or a plant comprising said cell. A protein of interest can be manufactured by culturing said cell, or growing a plant having said cell, etc. As used herein, "plant" encompasses individual plants, plant organs, plant tissues, plant cells, and seeds. Examples of plant organs include roots, leaves, stems, flowers, etc.

If the cell described above is a callus, the cell can be redifferentiated into a plant by a known redifferentiation method. For Oryza sativa, exemplary redifferentiation methods include the method described in Toki S. et al., Plant Physiol. 100(3): 1503-1507 (1992), the method described in Christou P. et al., Bio/Technology 9: 957-962 (1991), the method described in Hiei Y. et al., Plant J., 6: 271-282, (1994), etc.

Once a plant comprising a cell with a transformed organelle is obtained in this manner, progeny can be obtained by sexual or asexual reproduction from the plant. The plant can also be mass produced by obtaining a reproduction material (e.g., seeds, fruits, cut panicles, stubs, calluses, protoplasts, etc.) from the plant or progeny or clone thereof.

A protein of interest can also be isolated from cells cultured by the same method as the method described in 4. by extracting and purifying a useful component using a known method, or by allowing the protein of interest to be secreted in a culture and retrieving the protein. Alternatively, cells may be frozen and dried and used in such a state. Similarly, a protein of interest of a plant can be isolated by extracting and purifying the protein of interest as a useful component from the plant by a known method. Alternatively, a plant may be used directly. Examples of the known method described above include a method of crushing a plant with a mixer or mortar, then immersing the plant in water, saline, etc., and removing crushed residues by centrifugation or filtration.

### 6. Method of introducing an exogenous nucleic acid

Plant cells used in the screening method or manufacturing method of the invention can be manufactured by introducing an exogenous nucleic acid into an organelle of the plant cells. Thus, the screening method or manufacturing method of the invention may comprise the step of introducing an exogenous nucleic acid into an organelle of a plant cell.

An exogenous nucleic acid can be introduced into an organelle in a form of a vector having an ACC2 inhibitor resistant gene, spectinomycin resistant gene, compensating gene, and/or protein of interest encoding gene. It is preferable to add, to the vector described above, a sequence that is homologous to or has high identity to a sequence of a gene of an organellar DNA (e.g., trnG (tRNA-Gly (GCC)), trnV (tRNA-Val (GAC)), trnfM (tRNA-fMet (CAU)), rbcL gene, accD gene, trnI (tRNA-Ile (GAU)) and trnA (tRNA-Ala (UGU)), 3'rps12 (liposomal protein S12 exon-3) gene, trnV (tRNA-Val (GAC)), etc.), on the 5' and 3' sides of the introduced gene so as to be introduced into an organellar DNA by homologous recombination. Examples of the length of the homologous sequence include about 500 to 1500 bases. A homologous sequence can be appropriately designed based on base sequence information of an organellar DNA that is already known, etc.

The vector described above preferably has a gene described in a codon table in accordance with an organelle to be expressed. For example for vertebrates, a UGA codon means a stop codon in expression in the nucleus, but tryptophan is encoded in mitochondria. It is preferable that this is controlled so that a suitable protein sequence is expressed only in a suitable organelle in accordance with such a difference in the codon table.

It is preferable that an enhancer, promoter, leader and/or terminator sequence for regulating the expression of the gene described above is included upstream or downstream of the gene. For an organelle, a plurality of the genes may form an operon sequence. Examples of the enhancer include an enhancer region comprising a sequence upstream within a CaMV35S promoter, etc. Examples of the promoter include rrn promoter, cox2 promoter, psbA promoter, accD promoter, clpP promoter, atpB promoter, rpl32 promoter, H strand promoter, elongation factor 1α gene promoter (EF1α promoter), 35S promoter, PPDK promoter, PsPAL1 promoter, PAL promoter, UBIZM1 ubiquitin promoter, etc. In particular, an organelle derived promoter is preferred. Examples of the leader sequence include cry9Aa2 leader, atpB leader, etc. When inducing expression of a plurality of genes with one promoter, it is preferable to include a leader sequence. Examples of the terminator include rbcL terminator, rps16 terminator, CaMV35S terminator, ORF25polyA transcription terminator, psbA terminator, etc. In particular, an organelle derived terminator is preferred.

The vector described above can be constructed by, for example, introducing a nucleic acid with a gene of interest linked to the promoter described above into a suitable vector. Specific examples of a method thereof include, but are not limited to, the method described in Svab et al., Proc. Nal. Acad. Sci. USA, 87, 8526 (1990), the method described in Sikdar et al., Plant Cell Rep., 18, 20 (1998), the method described in Sidorov et al., Plant J., 19, 209 (1999), the method described in Ruf, S. et al., Nature biotechnol., 19, 870 (2001), the method described in Hou et al., Transgenic Res., 12, 111 (2003), etc.

Examples of a method of inserting a promoter, etc. into a vector include a method of cleaving a DNA comprising the promoter, etc. with a suitable restriction enzyme and inserting the DNA into a restriction enzyme site or multicloning site of a vector to link the promoter to the vector, etc.

A method of synthesizing the vector is not particularly limited. A conventionally known method can be employed. Examples of the synthesis method include a synthesis method through a genetic engineering approach, a chemical synthesis method, etc. Examples of a genetic engineering approach include an *in vitro* transcription synthesis method, method using a vector, and method using a PCR cassette. The vector is not particularly limited. Examples thereof include viral vectors, nonviral vectors such as a plasmid, etc. The chemical synthesis method is not particularly limited. Examples thereof include phosphoramidite method, H-phosphonate method, etc. The chemical synthesis method can use, for example, a commercially available automatic nucleic acid synthesizer. The chemical synthesis method generally uses amidite. The amidite is not particularly limited. Examples of commercially available amidite include ACE amidite, TOM amidite, CEE amidite, CEM amidite, TEM amidite, etc.

As a method of introducing the vector described above into an organelle of a cell for transformation, a known method, such as a particle gun method such as bombardment method (Svab Z., et al., Proc Natl acad Sci USA, 87: 8526-8530 (1990)), PEG method (Golds T., et al., Bio Technol., 11:95-97 (1992)), electroporation, etc. can be preferably used. For example in a particle gun method, a vector can be introduced into a cell by sprinkling a vector on very fine particles of gold or tungsten, and bombarding host cells with particles having the vectors adhering thereto using gun powder or high pressure gas. The definitions and types of organelles are described above in 1, but plastids or mitochondria are preferable, and chloroplasts or mitochondria in particular are more preferable. As described in Chuah J. A., et al., Scientific Reports, 5: 7751 (2015), it is preferable to introduce a vector into an organelle by using a complex of an organelle migration signal peptide and a cell membrane permeable peptide.

The present invention is described hereinafter through the Examples. However, the present invention is not limited to these Examples.

### [Examples]

### Example 1: Transformation of a chloroplast using a herbicide targeting a fatty acid biosynthesis system in monocotyledon Oryza sativa

### <Configuration of a target synthesis system>

The focus was placed on a biosynthesis system of fatty acid, which is a raw material of a lipid that is essential for the formation of a lipid bilayer such as a thylakoid membrane, as a lipid-soluble compound that is essential in a chloroplast. Many herbicides are known as a herbicide that targets and inhibits the key enzyme of a fatty acid biosynthesis system acetyl-CoA carboxylase (ACCase) of monocotyledon Poaceae, such as aryloxypropionate systems (aryloxyphenoxy-propionate; FOPs, clodinafop-propargyl, cyhalofop butyl, diclofop methyl, fenoxaprop-P-ethyl, fluazifop-P, haloxyfop R methyl, propaquizafop, quizalofop-P-ethyl, etc.), and cyclohexanedione systems (cyclohexanedione; DIMs, alloxydim, butroxydim, clethodim, profoxydim, sethoxydim, tepraloxydim, tralkoxydim, etc.), and phenylpyrazoline (DEN, pinoxaden) system. Meanwhile, it is known that a budding yeast derived ACC1 (ScACC1) retains the same enzymatic activity, but the function thereof is not inhibited by such herbicides. In this regard, a system for acquiring a chloroplast genome recombinant was configured by introducing a vector comprising ScACC1 into a chloroplast genome by homologous recombination while inhibiting fatty acid synthesis within a chloroplast with such herbicides.

### <Utilization of gene drive technology using a genome editing unit>

A phenomenon where the prevalence of a new genetic sequence that is introduced increases by including a genome editing unit or a part thereof in a genetic sequence to be introduced and configuring to cleave a sequence to be inserted of a genome before introduction with the genome editing unit upon introducing the new genetic sequence into a ploidy genome by homologous recombination is already known as a gene drive technology. However, an example of using this technology in a chloroplast does not exist. In general, protein or RNA transport remains within one organelle in organelle transformation. Thus, a method that effectively spreads these units requires an adjustment. In this case, a system expected to have an effect of gene drive within an organelle was configured by incorporating Cas9 with gRNA designating an expected introduction site on a chloroplast genome as a cleavage site into a new genetic sequence as a DNA sequence.

### <Construction of a plasmid>

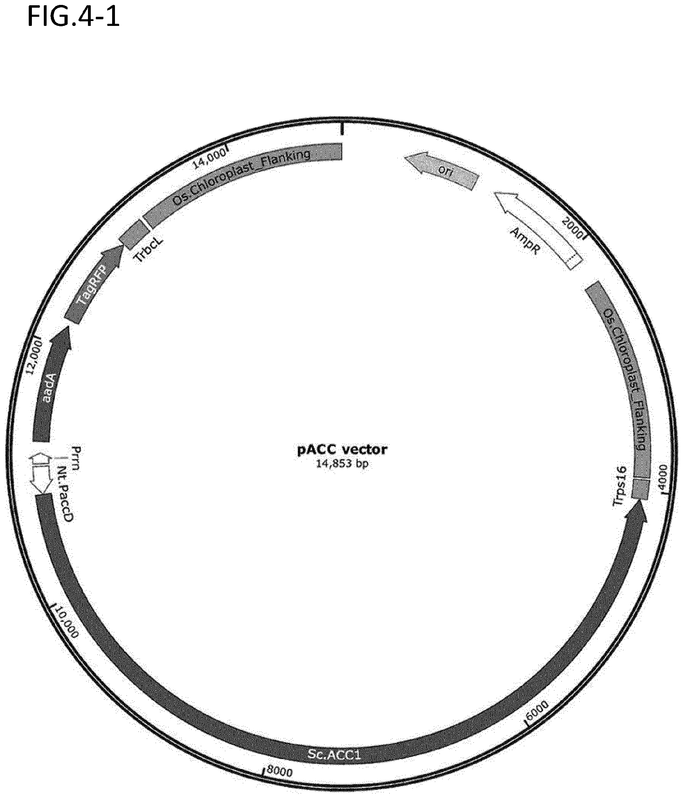

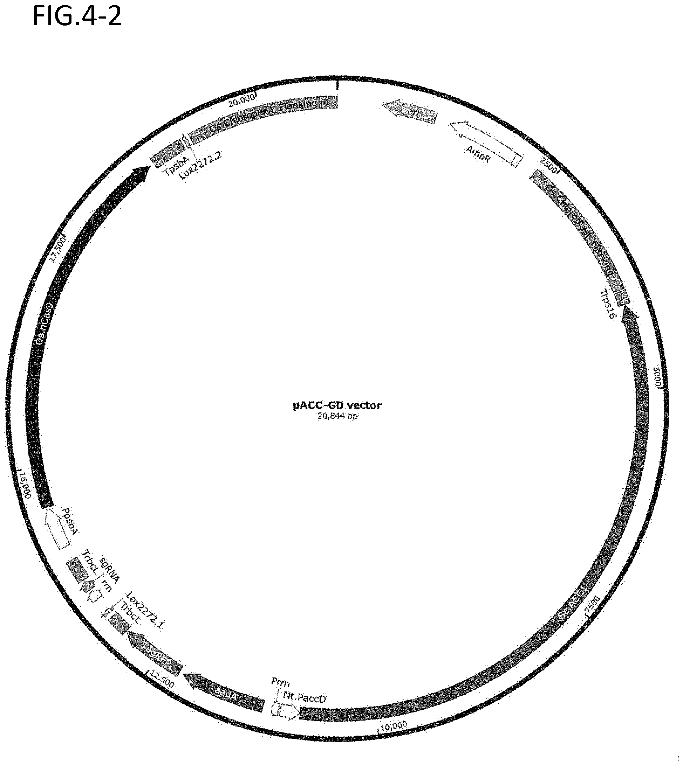

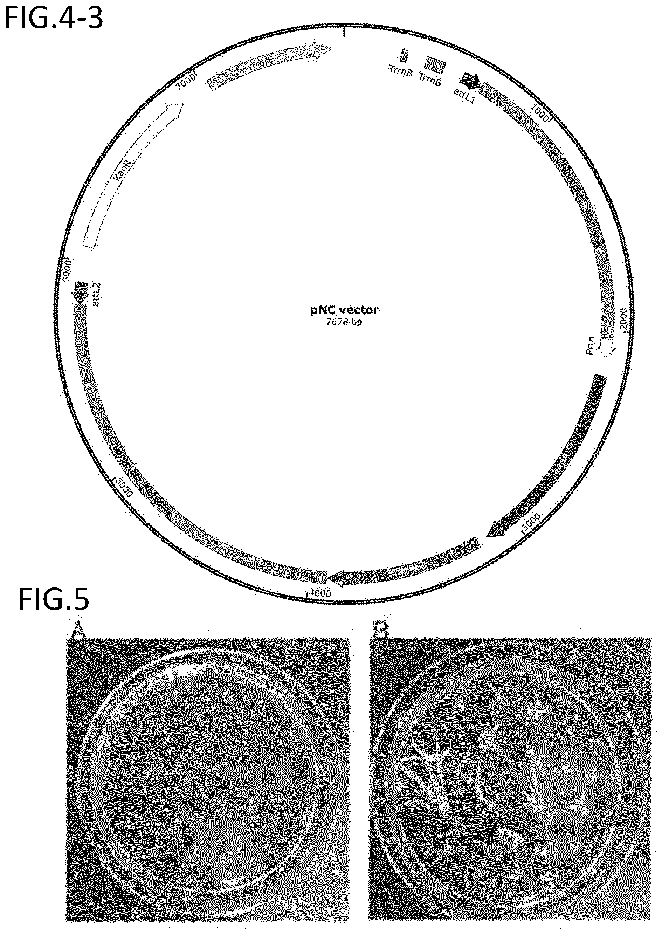

To investigate the chloroplast genome transformation technology described above, three types of vectors were constructed, i.e., pACC (Figure **4-1****)** (SEQ ID NO: 5) which only has ScACC1, pACC-GD (Figure **4-2****)** (SEQ ID NO: 6) which is further added with a genome editing unit, and pNC (Figure **4-3****)** (SEQ ID NO: 7) which does not have ScACC1 or a genome editing unit. Each vector was constructed to include a sequence homologous to a region to be inserted (trnI-trnA) on an Oryza sativa chloroplast genome and to flank a group of genes introduced with the trnI homologous sequence and trnA homologous sequence to incorporate the vector into the chloroplast genome by double crossover homologous recombination. A genome editing unit was configured to have sgRNA targeting a region to be inserted (Oryza sativa chloroplast genome trnI-trnA) and Os.nCas9 which is nickase (D10A) Cas9 with a codon optimized for Oryza sativa. For expression of each gene, a promoter that is not expressed within the nucleus but expected to be expressed in an organelle was selected, i.e., an accD promoter derived from Nicotiana tabacum chloroplast genome was selected for the expression of ScACC1, a psbA promoter derived from Oryza sativa chloroplast genome was selected for the expression of Os.nCas9, and an rrn promoter derived from Oryza sativa chloroplast genome was selected for the expression of sgRNA. A unit with a spectinomycin resistant gene (aadA) and red fluorescent protein (cgfTagRFP) gene disposed downstream of an rrn promoter derived from Nicotiana tabacum chloroplast genome was also introduced for all plasmids. Each constituent element of a plasmid was amplified by PCR to construct the plasmid by concurrently using ligation and NEBuilder HiFi DNA Assembly Master Kit (NEB).

<Culture conditions>

For the experiment, Oryza sativa japonica (Nipponbare) was used. Threshed Oryza sativa seeds were placed in a callus inducing medium, i.e., an N6D medium (Z. Shimatani et al. Nat Biol. 2017), and grown under conditions of 25°C away from light within an artificial climate incubator to induce a primary callus. To prepare a callus plate for introducing a gene, the primary callus that had been actively growing for several weeks since induction of the callus from seeds was strained using a sterilized mesh. The refined callus was uniformly applied on filter paper placed on a callus inducing medium plate. A gene was introduced to a plate obtained by culturing the callus for 3 to 4 days within the artificial climate incubator. For selection of a transformant, a redifferentiation medium which is an MSRE medium (Z. Shimatani et al. Nat Biol. 2017) added with a suitable herbicide was used and cultured under conditions of 31.5°C in a light-dark cycle of 12 hours within the artificial climate incubator. The callus was passaged once about every two weeks during culture in the redifferentiation medium.

### <Introduction of a plasmid DNA into a plant>

Plasmids of each of pACC, pACC-GD, and pNC were introduced to a prepared callus plate by bombardment. For bombardment, the apparatus PDS-1000/He (BioRad Laboratories) equipped with a Hepta adapter was used. The method of using the apparatus was in accordance with the protocol provided by BioRad Laboratories. As the conditions for introducing a gene, a DNA of the same mass per introduction to a callus plate was used, and a 1,100 p.s.i. rupture disk was used, with a placement of L3 so that the distance between a stopping screen and the callus plate would be 90 mm.

### <Selection of transformant>

After introduction of a gene, the plate was subjected to expansion culture under dark conditions in the artificial climate incubator for 3 to 4 days. After the expansion culture, callus particles were transferred to a redifferentiation selection medium with the filter paper. As the redifferentiation selection medium, a redifferentiation medium added with a herbicide was used. As the herbicide, haloxyfop belonging to FOPs or tralkoxydim belonging to DIMs was used at concentrations of 0.1, 0.5, 1.0, 2.0, or 4.0 µM. After introduction of a gene, the callus was passaged twice in the redifferentiation selection medium, then the callus was redifferentiated after about 6 weeks (Figure **5****).** The number of individuals redifferentiated from the callus clumps was counted as the selected individual count.

### <Detection of transgenesis in a created individual>

PCR and observation for fluorescence with a microscope were performed to detect the presence/absence of transgenesis in a created individual. A part of a shoot selected from a redifferentiation selection medium was harvested to extract the genomic DNA. The extracted genomic DNA was used as a template to identify an Oryza sativa chloroplast transformant by PCR. For detection of an Oryza sativa chloroplast recombinant, a primer targeting a sequence on (1) a selection marker ScACC1 gene (primer 1: CACTTCAATGTATTGATCGG (SEQ ID NO: 8), primer 2: TCGAGTCTTCTCCACAGAAGATGGAG (SEQ ID NO: 9)), (2) a bla gene that is present in the backbone region of a plasmid (primer 3: ATGCTGAAGATCAGTTGGGTGCACGAGTGG (SEQ ID NO: 10), primer 4: TTAATCAGTGAGGCACCTATCTCAGCGATC (SEQ ID NO: 11)), and (3) vicinity of an insertion region where a plasmid is incorporated in a nucleoid of an Oryza sativa chloroplast (primer 5: ATCGGAGTTATTTCCCAAGGACTTGCCATG (SEQ ID NO: 12), primer 6: CCGCCGACTCCAACTATCGTCCATGTACGA (SEQ ID NO: 13)) was designed to determine the presence/absence of amplification of a region of interest by PCR. If a plasmid is inserted into an Oryza sativa chloroplast, the region of (1) is amplified, and the regions of (2) and (3) are not amplified. If a plasmid remains in a plant, the regions of (1) and (2) are amplified, but if an Oryza sativa chloroplast recombinant is not present, only the region of (3) is specifically amplified and detected. The presence/absence of an amplified PCR fragment was determined by capturing a sample separated by 1.2% agarose electrophoresis and stained with GelRed by using a FAS3 gel imaging system. An inverted microscope Axio Observer 7 (Zeiss) was used for observation of fluorescence with a microscope. LSM800 (2xMA-PMT) was used for observation with a confocal microscope and capturing of microscope images.

### Results

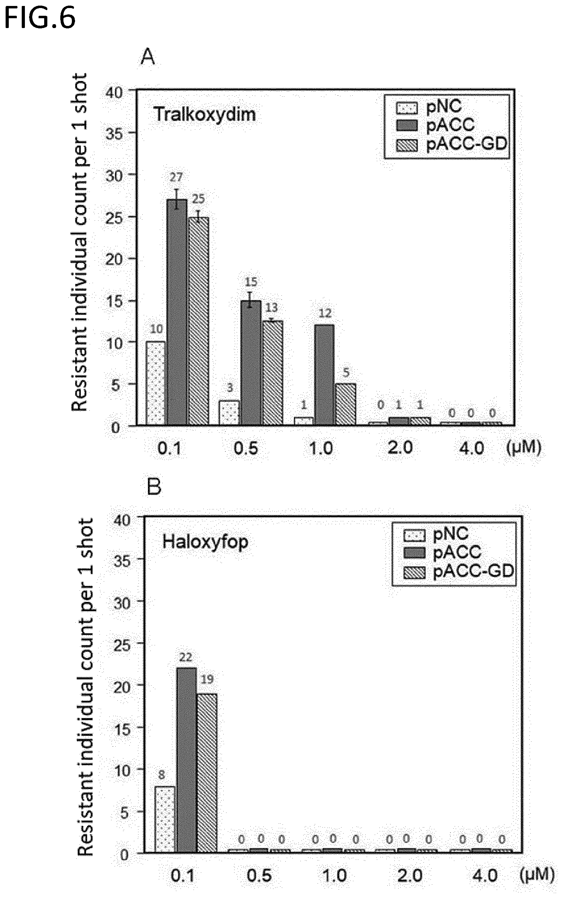

At the low herbicide concentration range, selected individuals were found even in a callus introduced with pNC, which does not impart resistance. It was revealed in view of the above that a selection effect of these herbicides can be expected at a concentration of 0.5 µM or greater for tralkoxydim (Figure **6A****)** and a concentration of 0.1 µM or greater for haloxyfop (Figure **6B****).** In fact, multiple selected individuals were found in callus introduced with pACC or pACC-GD at a herbicide sensitive concentration for pNC. This means that selection by pACC is functional. It was also found that suitable selection can be expected at a concentration range of 0.5 to 2 µM for tralkoxydim (Figure **6A****)** and 0.1 to 0.5 µM for haloxyfop (Figure **6B****)** under this experimental conditions, as the range in which the selection is functional. Furthermore, the number of selected individuals for pACC-GD was reduced by about 10 to 60% in all treatment areas compared to those of pACC. It is understood that this is because the vector size of pACC-GD is about 1.5-fold of the vector size of pACC (Figures **4-1** and **4-2),** so that the molecular weight of the DNA used upon transformation was lower (because the same mass of DNA was used in the protocol) .

Furthermore, to eliminate the possibility that these vectors merely impart a resistant phenotype by a transient expression, a test was conducted to check whether a vector sequence is incorporated into a chloroplast by PCR. As a result, a sequence of the backbone portion (portion eliminated upon homologous recombination) of a vector sequence was not amplified by PCR (Figure **7****).** Meanwhile, the portion of the introduced gene was amplified by PCR (Figure **7****).** These results suggest that a sequence expected to be introduced of a vector sequence was certainly incorporated into a chloroplast genome.

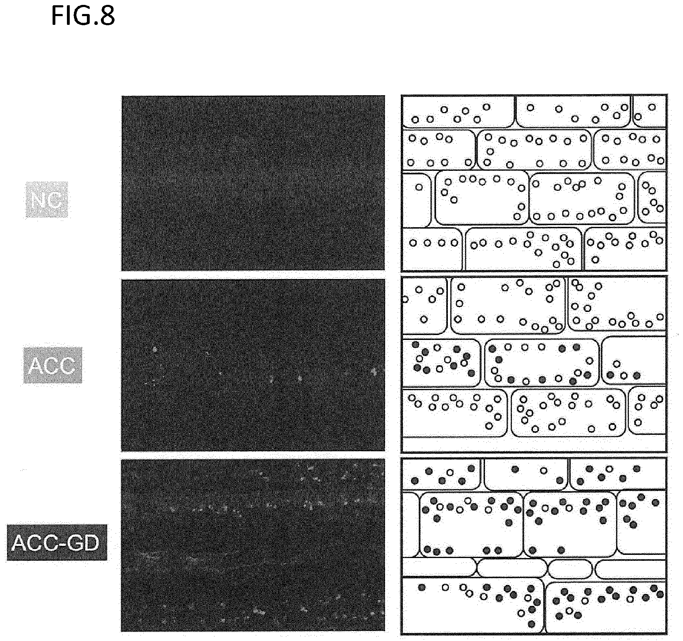

Since a TAgRFP expression cassette is incorporated into each vector, a transformed chloroplast can be detected by observing for TagRFP fluorescence. In this regard, when TagRFP fluorescence was observed with a confocal microscope, no fluorescence was observed for pNC, but fluorescence was observed for pACC and pACC-GD (Figure **8****).** This further confirms success of transformation into a chloroform genome described above. Furthermore, the ratio of transformed chloroplasts within a cell was higher for pACC-GD relative to pACC (Figure **8****).** As described above, the number of selected individuals that can be acquired was lower for pACC-GD than pACC, but it is suggested that efficiency greater than that of pACC can be expected from the viewpoint of homogenization of transformed chloroplast genomes in the acquired individuals.

### Example 2: Examination of a method of transforming a chloroplast using a herbicide targeting a fatty acid biosynthesis system in dicotyledon Arabidopsis thaliana

### <Configuration of a target synthesis system>

A group of herbicides targeting a fatty acid synthesis system in monocotyledon Oryza sativa examined in Example 1 is generally known as herbicides for monocotyledons. It is understood that this is because the enzyme responsible for ACCase localized in a chloroplast is different for monocotyledons and dicotyledons. Specifically, ACCase targeted by a group of herbicides encoded in the nucleus is responsible for fatty acid biosynthesis in a chloroplast in monocotyledons, whereas ACCase which is not targeted by a group of herbicides comprising a protein encoded by a chloroplast genome is responsible for fatty acid biosynthesis in a chloroplast in dicotyledons (it is known that ACCase of both types is responsible for fatty acid biosynthesis in a chloroplast in close relatives of Brassicaceae such as Arabidopsis thaliana used in this Example among dicotyledons) . In this regard, to expand the method in Example 1 to all dicotyledons, this Example tested the presence/absence of selection conditions for concerted action (not individual action) of the group of herbicides of Example 1 and spectinomycin that is used in chloroplast transformation of Nicotiana tabacum as a selection reagent and suppresses the expression of the entire protein encoded by a chloroplast in Arabidopsis thaliana.

### <Concerted sensitivity test on herbicide and antibiotic>

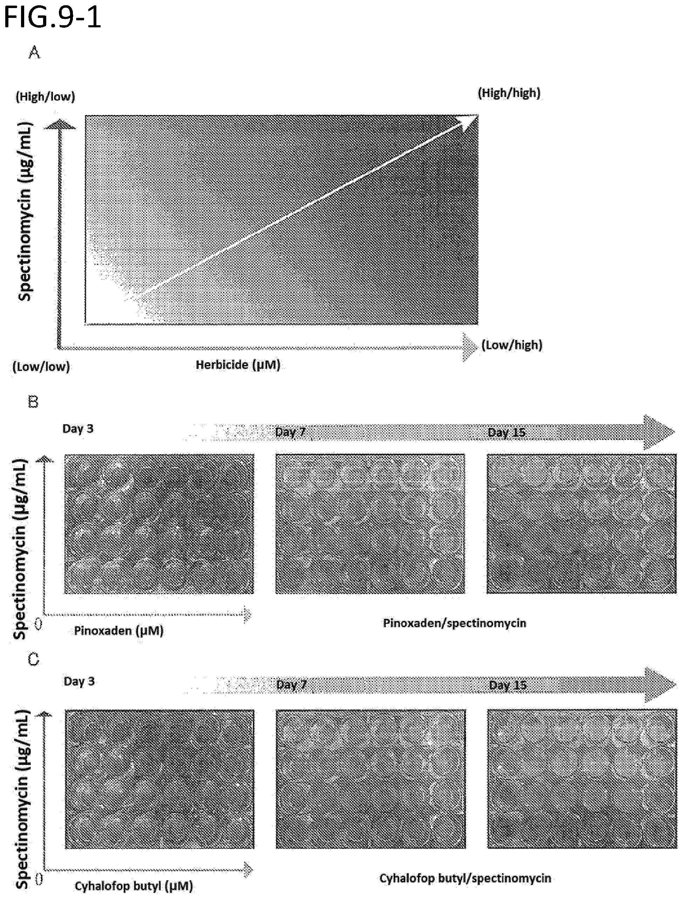

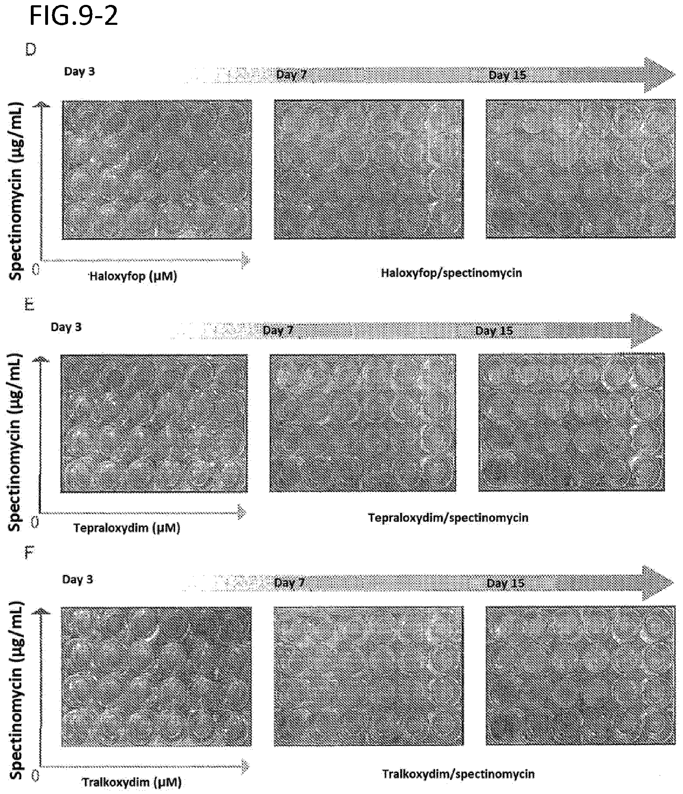

Arabidopsis thaliana cultured cells 87T were used in the experiment. Media comprising different concentrations of a group of herbicides and spectinomycin using a redifferentiation medium (Q. Yu. et al. Plant Physiol. 2017. 175(1). 186-193) as the base were added to each well of a 24-well plate. Specifically, a plate with varying concentrations of the group of herbicides of 0, 0.1, 1, 10, 50, and 100 µM in the direction of the X-axis of the plate and varying concentrations of an antibiotic spectinomycin of 0, 1, 10, and 100 ug/mL in the direction of the y-axis was prepared (Figure **9A).** Pinoxaden, tepraloxydim, tralkoxydim, cyhalofop butyl, and haloxyfop were used as the group of herbicides in each plate. 100 µL of cultured cells was added to each well and cultured under light-dark conditions in an artificial climate incubator. On days 3, 7, and 15 after culture, the growth status was checked to study the concerted sensitivity of a herbicide and antibiotic.

### Results

Change in color to yellow was confirmed under the condition of 100 ug/mL spectinomycin. This suggests that development of a chloroplast was obstructed. However, the growth rate of Arabidopsis thaliana was not inhibited with only spectinomycin within the concentration range studied in this experiment as already reported.

Meanwhile, inhibition of growth was observed under the conditions of 10 µM pinoxaden or greater and 50 µM haloxyfop or tralkoxydim or greater for the group of herbicides. Significant inhibition of the growth rate of Arabidopsis thaliana was not observed within the range of concentrations studied in this experiment for cyhalofop butyl and tepraloxydim.

Meanwhile, in the concerted sensitivity test group provided with both spectinomycin and the group of herbicides, significant inhibition of the growth rate, which was not observed in individual administration of the same concentration of pinoxaden, haloxyfop, tepraloxydim, or tralkoxydim, was confirmed. While it is expected that the specific selection conditions vary depending on the cell conditions or plant form, it was revealed that there is a concentration at which sensitivity concertedly changes in spectinomycin and the group of herbicides. The presence of such a condition suggests that chloroplast transformant can also be selected as in Example 1 in dicotyledons.

### Example 3: Chloroplast transformation using glycerolipid biosynthesis mutant

### <Configuration of a target lipid synthesis system and obtaining a mutant>

The focus was placed on a biosynthesis system of glycerolipid which is essential for the formation of a lipid bilayer such as a thylakoid membrane as a hydrophobic compound that is essential within a chloroplast. A pgp1-2 mutant which is an Arabidopsis thaliana strain with a gene encoding PGP1 that is a key enzyme for phosphatidyl glycerol (PG) synthesis thereamong destroyed was created (Hagio et al.) Since this mutant exhibits an albino phenotype, which is a dysfunctional trait of a chloroplast, by elimination of the PGP synthesis capability in a chloroplast, the mutant was obtained from the authors as the parent strain.

### <Construction of a plasmid>

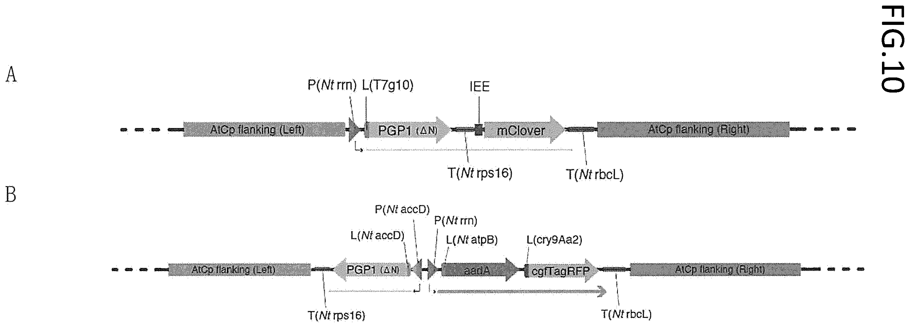

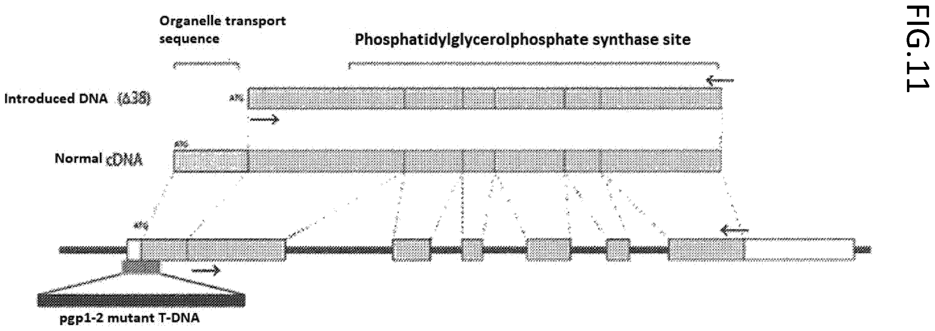

The following two vectors were used for the transformation of a chloroplast genome (Figure **10****).** The full length sequence of the vector (pKK4) shown in Figure **10A** is indicated as SEQ ID NO: 14, and the full length sequence of the vector (pKK5) shown in Figure **10B** is indicated as SEQ ID NO: 15. Sites homologous to the Arabidopsis thaliana chloroplast genome (NC_000932.1) were introduced at two locations on the left and right as homologous recombination sites to the chloroplast genome, and a gene to be introduced was placed therebetween. As shown in Figure **11****,** a chloroplast localization signal is encoded at the N-terminus of a protein sequence in a PGP1 gene used for selection. When introduced into a nucleus, PGP1 is transported to a chloroplast by using the chloroplast localization signal. By preparing a gene lacking this sequence portion, expression was expected only when this construct was introduced into a chloroplast. PGP1 gene cDNA lacking the N-terminus downstream of rrn, which is a known potent organelle promoter, and a fluorescent protein mClover gene were placed as genes to be introduced for pKK4. A spectinomycin resistant gene (aadA) and red fluorescent protein (cgfTagRFP) were placed downstream of rrn, which is a known potent organelle promoter, and PGP1 gene cDNA lacking an N-terminus was placed downstream of an accD promoter of Nicotiana tabacum as genes to be introduced for pKK5. Each vector was constructed by amplifying each constituent element by PCR by using NEBuilder HiFi DNA Assembly Master Kit (NEB).

<Introduction of plasmid DNA into a plant>

A plasmid was introduced into a chloroplast of a plant. A pgp1-2 mutant (Hagio et al.) of Arabidopsis thaliana was used as a plant and cultured in a Murashige & Skoog medium or soil. As the method of introducing a plasmid into a plant, a particle gun method for introducing DNA coated gold particles into a plant by pneumatic pressure or lipofection (Maeda et al. 2005, https://www.jstage.jst.go.jp/article/jibs/74/1/74_1_21/_art icle/-char/ja/) was used. For the particle gun method, Helios Gene Gun (Bio-Rad), PDS-1000/He (Bio-Rad), or a PDS-1000/He (Bio-Rad) device equipped with a Hepta adapter (Bio-Rad) was appropriately used. The method of using the device was in accordance with the protocol provided by Bio-Rad. A sugar-free medium was used for the selection of a transformant plant.

### <Detection of transgenesis in a created individual>

PCR or observation for fluorescence through a microscope was performed in order to detect the presence/absence of transgenesis in a created individual. PCR used DNA extracted from a created individual as a template DNA, the primer pair shown in Figure **11** (F: 5'-ATGTACTCCTCCCGTACATCTC-3' (SEQ ID NO: 16), R: 5'-CTACTTCATTAGTACTTTCCATATCTTC-3' (SEQ ID NO: 17)) as primers, and Quick Tag HS DyeMix (Toyobo) as a PCR enzyme. An amplified PCR product was subjected to agarose electrophoresis to check the size. An inverted microscope Axio Observer 7 (Zeiss) was used to observe fluorescence through a microscope, ZEISS Colibri 7 (Zeiss) was used as a light source, and 90HE (Zeiss) was used as a fluorescence filter to separate FITC fluorescence and TRITC fluorescence.

### Results

pKK4 was introduced into budding of a pgp1-2 mutant by the particle gun method. As a result, an albino phenotype with 100% turning yellow was exhibited without introduction of a DNA, but about 10% turned green in a pKK4 introduced line, as shown in Figure **12****.** This indicates that the pGP1 construct used in this experiment is transiently expressed in a chloroplast and can complement the phenotype of a pgp1-2 mutant. When a transformant was created by an introduction approach such as lipofection by using pKK5, which is expected to have a weaker expression of a PGP1 gene compared to pKK4, a green stable transformant was created as shown in Figure **13****.** Fluorescence believed to be emitted from TagRFP encoded by introduced DNA was confirmed in this plant through observation with a microscope. Furthermore, the plant was confirmed to highly retain the introduced DNA by PCR. It was confirmed in view of the above results that a chloroplast transformation system targeting a glycerolipid biosynthesis system functions as a hydrophobic compound that is essential in a chloroplast.

The descriptions in all of the documents mentioned herein including patents and patent applications are incorporated herein by reference to the same extent that the documents are explicitly disclosed in their entirety.

### [Industrial Applicability]

The present invention can efficiently transform an organelle in plant cells, especially monocotyledon cells or Brassicaceae plant cells, and can manufacture an organelle transformant by said method. Plant cells manufactured in this manner can create cells that stably and sufficiently express even a protein of interest that has an adverse effect on a host at maximum expression in an organelle.

The present application is based on Japanese Patent Application No. 2020-054225 (filing date: March 25, 2020) filed in the Japan. The entire content thereof is incorporated herein.

## Claims

1. A method of screening for monocotyledon cells with a transformed organelle, comprising the step of contacting an inhibitor of ACC2 with a cell population comprising monocotyledon cells introduced with a gene encoding acetyl-CoA carboxylase having resistance to the inhibitor and a gene encoding a protein of interest into an organelle.

2. The method of claim 1, wherein the acetyl-CoA carboxylase is derived from an organism other than the monocotyledon.

3. The method of claim 1 or 2, wherein the monocotyledon is a plant of the family Poaceae.

4. A method of selecting a plant cell with a transformed organelle, comprising the step of contacting an inhibitor of ACC2 and spectinomycin with a cell population comprising plant cells introduced with a gene encoding acetyl-CoA carboxylase having resistance to the inhibitor or a gene resistant to spectinomycin and a gene encoding a protein of interest into an organelle.

5. The method of any one of claims 1 to 4, wherein the inhibitor of ACC2 is at least one selected from the group consisting of pinoxaden, haloxyfop, tepraloxydim, and tralkoxydim.

6. The method of any one of claims 1 to 5, comprising the step of increasing a ratio of nucleoids having the introduced gene to nucleoids contained in an organelle by destroying nucleoids other than nucleoids having the introduced gene and selectively replicating nucleoids having the introduced gene, prior to contacting the inhibitor of ACC2 with plant cells.

7. The method of any one of claims 1 to 6, wherein the organelle is a plastid or a mitochondrion.

8. A plant cell screened for by the method of any one of claims 1 to 7 or a plant comprising said cell.

9. A method of manufacturing a plant cell with a transformed organelle, comprising the step of introducing a gene encoding a protein that compensates for a function of an enzyme involved with a synthesis pathway of an essential lipid-soluble compound into an organelle and a gene encoding a protein of interest to an organelle of a plant cell with the function of the enzyme that is attenuated.

10. The method of claim 9, wherein the enzyme involved with a synthesis pathway of an essential lipid-soluble compound is selected from the group consisting of PGP1, CLS, and MGD1.

11. A plant cell obtained by the method of claim 9 or 10, or a plant comprising said cell.
